# EUROPÄISCHE PATENTSCHRIFT

(11) **EP 0 424 801 B1**
(45) Veröffentlichungstag und Bekanntmachung des Hinweises auf die Patenterteilung: **24.11.1993**
(21) Anmeldenummer: 90119983.6
(22) Anmeldetag: 18.10.1990
(51) Int. Cl.: A01J 5/04, A01J 7/00, G01N 33/04

(54) **Vorrichtung zur Messung der elektrischen Leitfähigkeit von Milch**
Method for measuring the conductivity of milk
Procédé pour la mesure de la conductivité du lait

(30) Priorität: 27.10.1989 DE 3935759
(43) Veröffentlichungstag der Anmeldung: 02.05.1991
(73) Patentinhaber: LANG APPARATEBAU GMBH, 83313 Siegsdorf (DE)
(72) Erfinder: Wagner, Georg F., W-8240 Berchtesgaden (DE); Helminger, Karl, W-8229 Ainring (DE); Scheurl, Robert, W-8221 Inzell (DE); Wurm, Herbert, W-8227 Siegsdorf (DE)
(74) Vertreter: Wilk, Hans-Christof, Dr.

(56) Entgegenhaltungen:
- EP-A- 0 223 536
- DE-A- 3 308 361
- US-A- 3 566 841
- TRANSACTIONS OF THE ASEA, Band 27, Nr. 4, Juli/August 1984, Seiten 1204-1210, St. Joseph, Michigan, US; A.K. DATTA et al.: "Real time acquisition and analysis of milk conductivity data"

## Beschreibung

Die Erfindung richtet sich auf eine Meßkammer zur Messung der elektrischen Leitfähigkeit von während des Melkvorganges aus einer Zitze austretender Milch, bestehend aus einer im wesentlichen geschlossenen Meßkammer mit darin ausgebildeter, obenseitig offener Kammer mit eingelassener Meßzelle und einem auf die oberseitige Öffnung der Kammer und die Meßzelle ausgerichteten Milcheinlauf, der oberhalb der Kammer in einem oberseitigen Deckel der Meßkammer einmündend mit Abstand zu der Kammer endet, sowie einem außerhalb der Kammer und unterhalb von deren oberen Rand in der Wandung der Meßkammer angeordneten Milchablauf.

Es ist bekannt, daß die elektrische Leitfähigkeit der Milch von Kühen und ähnlichen Säugetieren bei der Entzündung von Drüsen im Zitzenbereich einen gegenüber dem gesunden Zustand des Tieres veränderten Wert aufweist. In dem Falle einer Entzündung liegt eine erhöhte elektrische Leitfähigkeit vor. Bei Kühen ist häufig nicht das ganze Euter, sondern nur eine Drüse von einer Entzündung befallen. Der Vergleich der elektrischen Leitfähigkeit der Milch zwischen den vier Drüsen sowie gegebenenfalls auch die Veränderung von Melkvorgang zu Melkvorgang gibt Aufschluß über den Zustand der Milch und über den Gesundheitszustand des Tieres. Durch die Messung der elektrischen Leitfähigkeit der Milch ist somit eine wirksame Methode gegeben, um subklinische Mastitis bei Kühen feststellen zu können.

Für das Melken von Kühen sind heute vielfach Melkmaschinen üblich, die mit einer Vakuumpulsation arbeiten und mittels dieses Vorganges die Zitze bzw. Zitzen auspressen. Diese Vakuumpulsation bewirkt einen die Richtung wechselnden Luftstrom und das Absaugen der Milch in ein Vorratsgefäß.

Während des Absaugvorganges entsteht in derartigen Melkmaschinen eine turbulente Strömung im Rohr und im Melkgeschirr. Außerdem entstehen Milchschaum und Luftblasen die für eine herkömmliche On-line-Messung der elektrischen Leitfähigkeit schädlich sind. Setzt man in derartigen Melkmaschinen übliche Elektroden aus Stahl oder Edelmetall in eine Rohrleitung des Melkgeschirrs ein, so erhält man einen stark schwankenden Meßwert, der sich zudem noch durch Veränderungen der Elektrodenoberfläche durch Fett, Verschmutzung und ähnliche Vorgänge verändert. Schließlich wird bei einem derartigen bekannten Vorgehen noch durch den Neigungswinkel der Meßvorrichtung das Meßergebnis mitbestimmt bzw. sogar regelmäßig verfälscht.

Eine gattungsgemäße Meßkammer ist aus der DE-A-33 08 361 bekannt. Diese vorbekannte Meßkammer weist zur Schaffung eines beruhigten Probestromes bereits eine beruhigte Strömungszone auf. Der Milcheinlauf ist in axialer Fortsetzung oberhalb einer in der Meßkammer ausgebildeten Kammer angeordnet, die an ihrem Boden eine Meßzelle aufweist. Auch bei dieser vorbekannten Meßkammer besteht noch immer das Problem, daß die Milch schäumt und stark verwirbelt wird. Dieses Problem besteht auch bei einer Meßkammer, die in TRANSACTIONS OF THE ASEA, Band 27, Nummer 4, Juli/August 1984, Seiten 1204 bis 1210, A.K. Datta et al. beschrieben ist. Auch bei dieser Meßkammer wird der Pulsationsstrom durch die Meßzelle und an der Meßelektrode vorbeigeführt.

Aufgabe der Erfindung ist die Schaffung einer Lösung, die in Gegenwart von Luft und Vakuumpulsationströmungen die Messung des elektrischen Leitwertes eines oszillierenden Milchstromes in einem Milchgeschirr verbessert.

Bei einer Meßkammer der eingangs bezeichneten Art wird diese Aufgabe gemäß der Erfindung dadurch gelöst, daß die Kammer ein in die Meßkammer eingesetztes, oberseitig offenes, topfartiges Gehäuse mit darin angeordneter induktiver Meßzelle und Temperaturfühler ist, wobei die Meßzelle ein Ringkörper ist und eine Erregerspule und eine dazu koaxial angeordnete Empfängerspule aufweist, und daß der Milcheinlauf und der Milchablauf sowie das Gehäuse derart ausgelegt sind, daß eine Luftströmung direkt vom Milcheinlauf an dem topfartigen Gehäuse vorbei zum Milchablauf gelangt.

Durch die Erfindung ist es möglich, den elektrischen Leitwert der Milch auch bei Vakuumpulsationsströmen exakt und reproduzierbar zu messen. Es wird mit einer induktiven, d.h. elektrodenlosen, Meßzelle gearbeitet, so daß die Meßkammer verschmutzungs- und verfettungsunempfindlich ist und einen permanenten Meßwert liefert. Des weiteren ist die Meßzelle in einem topfartigen Gehäuse angeordnet, welches sich innerhalb einer geschlossenen Meßkammer befindet. Die Meßkammer weist einen Zulauf auf, durch welchen die Milch unmittelbar nach dem Austritt aus einer Zitze in die Meßkammer gelangt. Dieser Zulauf weist einen vertikalen Abstand zum oberen Rand des topfartigen Gehäuses mit der Meßzelle auf. Weiterhin ist diese Anordnung bzw. Zuordnung von Milcheinlauf und Milchablauf sowie dem topfartigen Gehäuse so ausgelegt, daß etwaige Vakuumpulsations-Strömungen und Luftströmungen direkt vom Milcheinlauf an dem topfartigen Gehäuse vorbei zum Milchablauf gelangen. Durch diese Konstruktion wird eine Beeinflussung der elektrischen Leitwertmessung durch die Vakuumpulsation ausgeschaltet. Weder wird die Milch aufgeschäumt noch der Meßtopf leergeblasen. Aufgrund der Anordnung und Ausbildung von Milchzulauf und Milchablauf außerhalb des Meßtopfes wird eine Vorbeileitung des Vakuumpulsationsluftstromes an der Meßstelle bewirkt, so daß in der Meßzelle ein Meßwert für die Milch gemessen wird, der ohne größere Probleme mit Hilfe einer Meßwertelektronik ausgewertet werden kann. Im Gegensatz zu bekannten Elektrodensystemen in Rohrleitungen ist es bei Verwendung der Erfindung nicht mehr notwendig, sogenannte Hüllkurven zu bilden, sowie auch nicht mehr notwendig mit Unsicherheitsfaktoren im Hinblick auf die Erreichung eines sogenannten wahren Spitzenwertes zu rechnen.

Mit der Erfindung ist es möglich Mastitis bei Kühen zuverlässig zu erkennen. Da Milch aus einer mit Mastitis befallenen Zitze einen teilweise nur geringfügig und nur kurzzeitig erhöhten Leitwert aufweist, war die Erkennung der Signifikanz in der Praxis unter Betriebsbedingungen mit Vorrichtungen und Verfahren nach dem Stand der Technik nicht zuverlässig möglich. Mit der Erfindung bereitet dies aber auch unter teilweise ungünstigen Betriebsbedingungen keine Probleme.

Schließlich ist die erfindungsgemäße Vorrichtung in weiten Neigungsbereichen der Meßvorrichtung anwendbar sowie weitgehend unempfindlich gegenüber Bewegung und Erschütterungen.

Weitere Vorteile ergeben sich aus den Unteransprüchen.

Dadurch, daß dem Meßtopf und damit der Meßzelle ein Temperaturfühler zugeordnet ist, ist die Milchtemperatur unmittelbar nach dem Verlassen der Zitze schnell und richtig zu bestimmen. Wenn die Vorrichtung aus vier getrennt arbeitenden Meßsystemen, die jeweils von der Milch aus einer Zitze durchströmt werden, besteht, ist es möglich, den elektrischen Leitwert der Milch aus jeder einzelnen Zitze getrennt zu bestimmen. Dasselbe gilt natürlich für die Temperatur. Erst anschließend wird die Milch im Melkgeschirr gesammelt, vermischt und über eine Rohrleitung einem Vorratsgefäß zugeführt. Hierdurch ist es möglich, zunächst festzustellen, ob z.B. nur eine Zitze bzw. Drüse erkrankt ist, um dann daraufhin die Milch aus dieser einen Drüse bzw. Zitze zu separieren und die übrige, aus den gesunden Zitzen austretende Milch einer Verwertung zuzuführen. Dies kann insbesondere im Rahmen zukünftiger Milchabgabe-Verordnungen von Wichtigkeit sein, da beabsichtigt ist, das Milchgeld an den Landwirtschaftsbetrieb unter anderem von der Qualität der Milch (keimfrei, Leitwert) abhängig zu machen.

Die Erfindung ist nachstehend anhand der Zeichnungen beispielsweise näher erläutert. Diese zeigt in
- Figur 1: einen Querschnitt durch eine erfindungsgemäße Vorrichtung,
- Figur 2: einen Schnitt längs der Linie II/II in Figur 1 und in
- Figur 3: eine Aufsicht auf eine Anordnung von vier erfindungsgemäßen Vorrichtungen

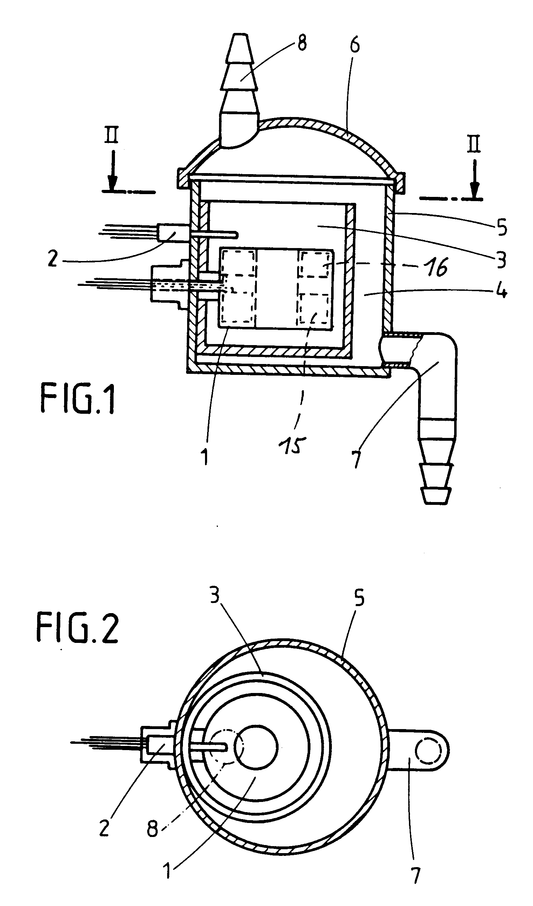

Die Figur 1 zeigt eine kreisringförmig ausgebildete induktive Meßzelle 1 sowie einen Temperaturfühler 2. Die induktive Meßzelle 1 und der Temperaturfühler 2 sind in einem topfartigen Gehäuse 3 aus Kunststoff angeordnet. Das topfartige Gehäuse 3 bzw. dieser Meßtopf seinerseits ist wiederum in einer insgesamt mit 4 bezeichneten Meßkammer angeordnet, die aus einem Meßkammerunterteil 5 und einem Meßkammeroberteil 6 besteht. Das topfartige Gehäuse 3 liegt mit einem Teil seiner Außenwandung an der Innenwand der Meßkammer 4 an. Im Bereich dieser Anlageflächen treten die Meßleitungen bzw. Anschlüsse von Meßzelle 1 und Temperaturfühler 2 durch die Wände bzw. Wandungen von Meßtopf 3 und Meßkammer 4 hindurch nach außen. Die Meßzelle 1 besteht aus einer Erregerspule 15 und einer hierzu coaxialen Empfängerspule 16 oder Sekundärspule. Die beiden Spulen 15 und 16 bilden einen eisenlosen Transformator. Die Erregerspule 15 wird von einem Oszillator mit einer Erregerfrequenz von ca. 5 bis 10 kHz gespeist. In Abhängigkeit von dem Leitwert der im Inneren der Spulen befindlichen Milch, wie nachstehend erläutert, entsteht an der Empfängerspule 16 eine Spannung, die von einem angeschlossenen Verstärker (nicht dargestellt) verstärkt wird. Diese Spannung bildet das Leitfähigkeitssignal.

Vorzugsweise sind das Meßkammerunterteil 5 und das topfartige Gehäuse 3 querschnittlich zylindrisch ausgebildet, aber exzentrisch zueinander angeordnet. Sowohl die Grundfläche als auch das umfaßte Volumen des topfartigen Gehäuses 3 sind deutlich kleiner als das der Meßkammer 4. Das Meßkammeroberteil 6 ist als Deckel zum Meßkammerunterteil 5 ausgebildet und weist einen Zulauf 8 für von einer Zitze abgesaugte Milch auf. Die Mündung des Zulaufes 8 in die Meßkammer 4 weist einen vertikalen Abstand zum oberen Rand des topfartigen Gehäuses 3 auf. Von dieser Einmündung fließt die Milch direkt in den Meßtopf 3 mit darin befindlichem Temperaturfühler 2 und Meßzelle 1. Ein Ablauf 7 für die Milch ist außerhalb und unterhalb des oberen Randes des Meßtopfes 3 in der Seitenwand der Meßkammer 4 ausgebildet. Die durch den Einlauf 8 zulaufende Milch sammelt sich solange in dem topfartigen Gehause 3 bis sie über dessen oberen Rand überläuft und dann durch den Aus lauf 7 aus der Meßkammer 4 wieder austritt. Da die Meßkammer 4 dafür vorgesehen ist, in Melkmaschinen angeordnet zu sein, die den Melkprozeß mit Hilfe einer Vakuumpulsation betreiben, ist die vorstehend beschriebene Anordnung bzw. Zuordnung von Einlauf 8, topfartigem Gefäß 3 und Ablauf 7 so ausgelegt, daß etwaige Vakuumpulsationsströmungen und Luftströmungen direkt vom Einlauf 8 an dem topfartigen Gehäuse 3 vorbei zum Aus lauf 7 gelangen. Hierdurch wird verhindert, daß die Meßwerterfassung mittels der Meßzelle 1 in dem Meßtopf 3 durch Luft oder ein dadurch bewirktes Aufschäumen der Milch beeinflußt und beeinträchtigt wird.

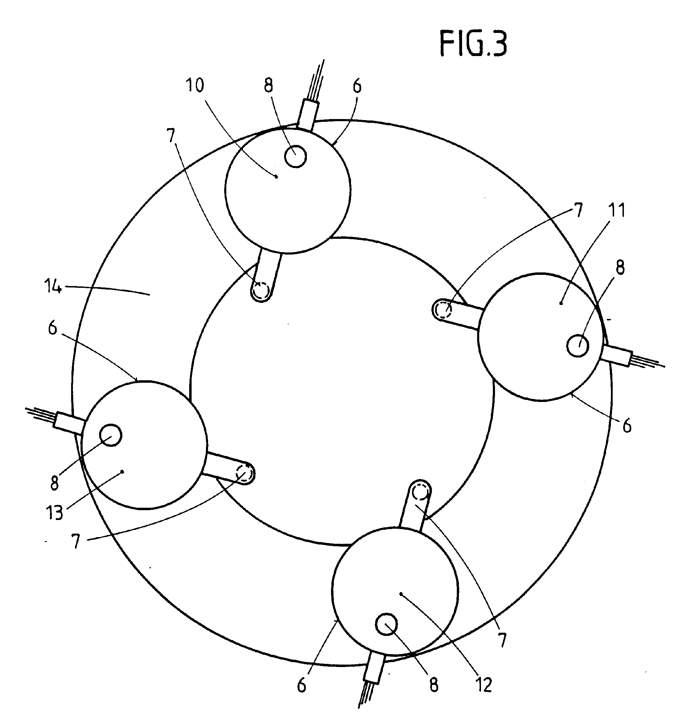

Die Figur 3 zeigt eine Draufsicht auf vier an einem kreisringförmigen Halter 14 befestigte Meßkammern 10, 11, 12 und 13. Die übrigen Bezugszeichen entsprechen denen der Figuren 1 und 2. Diese Anordnung ist dafür ausgelegt, die elektrischen Leitwerte der Milch, die aus den vier Zitzen eines Kuheuters austritt, getrennt zu erfassen.

In der Regel sind Meßkammern 4 zwischen Zitzenbecher und Milchsammler vorzugsweise in die Gummiteile der einzelnen Zitzenbecher installiert. Die einzelnen Meßkammern 4 weisen eine Bauhöhe von ca. 50 bis 60 mm auf und haben ein Gewicht im Leerzustand von ca 100 bis 150 g. Vorhandenes Melkgeschirr kann mit derartigen Meßkammern nachgerüstet werden. Es ist aber auch möglich, die Meßkammern 4, vorzugsweise in einer miniaturisierten Ausführungsform, direkt in den Michsammler zu integrieren. In diesem Fall besteht der Milchsammler vorzugsweise aus Kunststoff. Die induktive Meßzelle wird mit einer Schutzkleinspannung bzw. Niedrigspannung betrieben, die für Tier und Mensch ungefährlich ist. Die Auswertung der hier erhaltenen Meßsignale erfolgt über übliche Meßwertumformer, mittels welcher die Meßwerte registriert oder für eine Weiterverarbeitung aufbereitet werden.

Insgesamt gesehen ist die Vorrichtung, insbesondere die nach Figur 3, derart in einem Melkgeschirr bzw. einer Melkmaschine eingebaut und angeordnet, daß jede einzelne Zitze während des Melkvorganges gemessen, d.h. der elektrische Leitwert sowie die Temperatur der aus der jeweiligen Zitze austretenden Milch getrennt gemessen werden kann. Unmittelbar nach dem Verlassen der Zitze strömt die Milch durch den Einlauf 8 in das topfartige Gehäuse 3 der Meßkammer 4, wo mittels der induktiven Meßzelle 1 der elektrische Leitwert gemessen wird. Durch Überlaufen über den oberen Rand des Meßtopfes 3 gelangt die Milch dann in den Ablauf 7 wo sie wie üblich abgesaugt wird. In dem Meßtopf arbeitet die induktive Leitfähigkeitsmeßeinrichtung unabhängig von der Verfettung und Verschmutzung sowie kleinen Luftbläschen in der Milch. Der Meßtopf 3 ist in der Meßkammer 4 so eingebaut, daß die Vakuumpulsation zwischen der Melkeinrichtung, d.h. dem Milchgeschirr oder dem Milchsammler und dem Zitzenbecher ohne Störung der laufenden Leitwertmessung strömen kann. Auch der Überlauf der Milch aus dem Meßtopf 3 in die Meßkammer 4 stört aufgrund der konstruktiven Anordnung das Meßergebnis nicht. Weiterhin ist eine "Schiefstellung" der Meßkammer 4 bis zu einem Neigungswinkel von ca. 30° unschädlich für das Meßergebnis.

Der Meßtopf 3 kann weiterhin im Boden eine Bohrung mit einem Durchmesser von 1 - 2 mm aufweisen. Durch diese Bohrung kann in dem Meßtopf 3 befindliche Milch langsam in die Meßkammer 4 auslaufen. Da diese Bohrung allerdings einen Nebenschluß zum induktiven Meßsystem im Meßtopf 3 darstellt, ist in diesem Fall die Veränderung der Meßzellenkonstante bei der Auswertung des Meßergebnisses zu berücksichtigen.

## Patentansprüche

1. Meßkammer (4) zur Messung der elektrischen Leitfähigkeit von während des Melkvorganges aus einer Zitze austretender Milch, bestehend aus einer im wesentlichen geschlossenen Meßkammer (4) mit darin ausgebildeter, obenseitig offener Kammer mit eingelassener Meßzelle (1) und einem auf die oberseitige Öffnung der Kammer und die Meßzelle (1) ausgerichteten Milcheinlauf (8), der oberhalb der Kammer in einem oberseitigen Deckel (6) der Meßkammer (4) einmündend mit Abstand zu der Kammer endet, sowie einem außerhalb der Kammer und unterhalb von deren oberen Rand in der Wandung der Meßkammer (4) angeordneten Milchablauf (7),
dadurch gekennzeichnet,
daß die Kammer ein in die Meßkammer (4) eingesetztes, oberseitig offenes, topfartiges Gehäuse (3) mit darin angeordneter induktiver Meßzelle (1) und Temperaturfühler (2) ist, wobei die Meßzelle (1) ein Ringkörper ist und eine Erregerspule (15) und eine dazu koaxial angeordnete Empfängerspule (16) aufweist, und daß der Milcheinlauf (8) und der Milchablauf (7) sowie das Gehäuse (3) derart ausgelegt sind, daß eine Luftströmung direkt vom Milcheinlauf (8) an dem topfartigen Gehäuse (3) vorbei zum Milchablauf (7) gelangt.

2. Vorrichtung nach Anspruch 1,
dadurch gekennzeichnet,
daß das topfartige Gehäuse (3) und die Meßkammer (4) mit Wandungsbereichen aneinanderliegen, in welchen Durchtrittsöffnungen für Bereiche des Temperaturfühlers (2) und der Meßzelle (1) ausgebildet sind.

3. Vorrichtung nach Anspruch 1 oder 2,
dadurch gekennzeichnet,
daß die Meßkammer (4) in der Melkvorrichtung zwischen Zitzenbecher, insbesondere dessen üblichen Gummiteilen, und dem Melkgeschirr, insbesondere dem Milchsammler, installiert ist.

4. Vorrichtung nach einem der vorangehenden Ansprüche,
dadurch gekennzeichnet,
daß die Meßkammer in einen Milchsammler, vorzugsweise aus Kunststoff, integriert ist.

5. Vorrichtung nach einem der vorangehenden Ansprüche,
dadurch gekennzeichnet,
daß die Meßkammer (4) eine Höhe von 50 bis 60 mm und ein Gewicht von 100 bis 150 g aufweist.

6. Vorrichtung nach einem der vorangehenden Ansprüche,
dadurch gekennzeichnet,
daß der Meßzelle (1) ein Meßwertumformer zur Auswertung der Meßsignale zugeordnet ist.

7. Vorrichtung nach einem der vorangehenden Ansprüche,
dadurch gekennzeichnet,
daß vier Meßkammern (10, 11, 12, 13), für jeden Zitzenbecher eine, in einem Melkgeschirr angeordnet sind.

8. Vorrichtung nach Anspruch 7,
dadurch gekennzeichnet,
daß die Meßkammern (10, 11, 12, 13) auf einem kreisringförmigen Halter (14) angeordnet sind.

## Claims

1. Measuring chamber (4) for the measurement of the electrical conductivity of milk issuing from a teat during the milking operation and consisting of a substantially closed measuring chamber (4) with an upwardly open chamber formed therein with a let-in measuring cell (1) and a milk inlet (8), which is oriented towards the upward opening of the chamber and the measuring cell (1) and which ends above the chamber and at a spacing from the chamber while opening into an upper lid (6) of the measuring chamber (4), as well as a milk outlet (7), which is arranged within the wall of the measuring chamber (4) outside the chamber and underneath the upper rim thereof, characterised thereby, that the chamber is an upwardly open potlike housing (3), which is inserted into the measuring chamber (4), with an inductive measuring cell (1) and a temperature sensor (2) arranged therein, wherein the measuring cell (1) is an annular body and displays an exciter coil (15) and a receiver coil (16) arranged co-axially thereto, and that the milk inlet (8) and the milk outlet (7) as well as the housing (3) are designed in such a manner that an air flow gets directly from the milk inlet (8) past the potlike housing (3) to the milk outlet (7).

2. Device according to claim 1, characterised thereby, that the potlike housing (3) and the measuring chamber (4) lie one against the other by wall regions, in which passage openings are formed for regions of the temperature sensor (2) and the measuring cell (1).

3. Device according to claim 1 or 2, characterised thereby, that the measuring chamber (4) is installed in the milking device between the teat cup, in particular its customary rubber parts, and the milking harness, in particular the milk collector.

4. Device according to one of the preceding claims, characterised thereby, that the measuring chamber (4) is integrated into a milk collector which is preferably of synthetic material.

5. Device according to one of the preceding claims, characterised thereby, that the measuring chamber (4) displays a height of 50 to 60 millimetres and a weight of 100 to 150 grams.

6. Device according to one of the preceding claims, characterised thereby, that the measuring cell (1) is associated with a measurement value transformer for evaluation of the measurement signals.

7. Device according to one of the preceding claims, characterised thereby, that four measuring chambers (10, 11, 12, 13), one for each teat cup, are arranged in a milking harness.

8. Device according to claim 7, characterised thereby, that the measuring chambers (10, 11, 12, 13) are arranged on a holder (14) in the shape of a circular ring.

## Revendications

1. Chambre de mesure (4) pour la mesure de la conductivité électrique du lait sortant d'un trayon au cours du processus de la traite, constituée par une chambre de mesure (4) complètement fermée dans laquelle est réalisée une chambre interne ouverte sur son côté supérieur, pourvue d'une cellule de mesure (1) encastrée et d'une cellule d'admission (8) pour le lait située à l'aplomb de l'ouverture du côté supérieur de la chambre inteme et de la cellule de mesure (1) et dont l'extrémité inférieur débouche, à distance de la chambre interne, dans un couvercle (6) situé sur la partie supérieure de la chambre de mesure (4) et au-dessus de la chambre interne, ainsi que d'une évacuation (7) du lait prévue dans la paroi de la chambre de mesure (4) au-dessus de la chambre interne et en-dessous du bord supérieur de celle-ci, cette chambre de mesure étant caractérisée par le fait que la chambre interne est constituée par une cage (3) du genre pot, ouverte sur son côté supérieur, logée dans la chambre de mesure (4) et incorporant une cellule inductrice de mesure (1) et un sondeur de température (2), la cellule de mesure (1) étant constituée par un corps annulaire et étant pourvue d'une bobine excitatrice (15) et d'une bobine réceptrice (16) disposée de manière coaxiale à cette dernière, et par le fait que l'admission (8) et l'évacuation (7) pour le lait, ainsi que la cage (3), sont étudiés de manière qu'un courant d'air parvient directement de l'admission (8) pour le lait jusqu'à l'évacuation pour celui-ci (7) en passant près de la cage (3) du genre pot.

2. Dispositif selon la revendication 1, caractérisé par le fait que la cage (3) du genre pot et la chambre de mesure (4) sont contigues par des parties de leurs parois, dans lesquelles des ouvertures de passage sont prévues pour des zones du sondeur de température (2) et de la cellule de mesure (1).

3. Dispositif selon l'une ou l'autre des revendications 1 ou 2, caractérisé par le fait que la chambre de mesure (4) est installée, dans le dispositif de traite, entre le gobelet trayeur, en particulier les parties usuelles en caoutchouc de celui-ci, et le récipient à traire, en particulier le collecteur de lait.

4. Dispositif selon l'une quelconque des revendications 1 à 3, caractérisé par le fait que la chambre de mesure (4) est intégrée dans un collecteur de lait constitué de préférence en matière plastique.

5. Dispositif selon l'une quelconque des revendications 1 à 4, caractérisé par le fait que la chambre de mesure (4) a une hauteur de 50 à 60 mm et un poids de 100 à 150 g.

6. Dispositif selon l'une quelconque des revendications 1 à 5, caractérisé par le fait qu'un convertisseur de signaux coopère avec la cellule de mesure (1) pour l'analyse des signaux de mesure.

7. Dispositif selon l'une quelconque des revendications 1 à 6, caractérisé par le fait que quatre chambres de mesure (10, 11, 12, 13) - une pour chaque gobelet trayeur - sont disposées dans un récipient à traire.

8. Dispositif selon la revendication 7, caractérisé par le fait que les chambres de mesure (10, 11, 12, 13) sont placées sur un support (14) en forme de couronne.
